# EUROPÄISCHE PATENTSCHRIFT

(11) **EP 1 340 470 B1**
(45) Veröffentlichungstag und Bekanntmachung des Hinweises auf die Patenterteilung: **15.09.2004**
(21) Anmeldenummer: 02004345.1
(22) Anmeldetag: 01.03.2002
(51) Int. Cl.: A61B 19/00, F21S 8/00

(54) **Operationslampe mit Kamerasystem zur 3D-Referenzierung**
Operation theatre lighting device with camera system for three-dimensional referencing
Lampe pour salle d'opération comportant un système de caméras pour référencement tri-dimensionnel

(43) Veröffentlichungstag der Anmeldung: 03.09.2003
(73) Patentinhaber: BrainLAB AG, 85551 Kirchheim/Heimstetten (DE)
(72) Erfinder: Bauch, Thomas, 80469 München (DE); Vilsmeier, Stefan, 6330 Kufstein (AT)
(74) Vertreter: Schwabe - Sandmair - Marx

(56) Entgegenhaltungen:
- EP-A- 0 421 130
- EP-A- 0 793 945
- WO-A-01/05161
- WO-A-99/27839
- US-A- 5 808 680
- US-B1- 6 246 900
- US-B1- 6 309 345

## Beschreibung

Die vorliegende Erfindung betrifft ein System zum kombinierten schattenfreien Beleuchten eines vorgebbaren Bereichs und zur Referenzierung von dreidimensionalen Raumkoordinaten, insbesondere von chirurgischen oder medizinischen Instrumenten, sowie ein Reflektorenreferenzierungssystem.

Referenzierungssysteme im Sinne dieser Anmeldung sind beispielsweise aus der DE 196 39 615 C2 bekannt. Solche auch als Navigationssysteme bekannten Systeme stellen die Verbindung zwischen den behandelnden Chirurgen, d. h. der Patientenanatomie, wie sie der Chirurg bei der Behandlung sieht, und diagnostischen Daten her, die beispielsweise durch eine Computertomographie erhalten wurden und durch eine Rechnereinheit mit einer Bildausgabe visuell dargestellt werden. Zur Erfassung dreidimensionaler Raumkoordinaten werden eine Mehrzahl von Markern mit bekannten Abmessungen eingesetzt, wie beispielsweise in der DE 196 39 615 C2 Zeile 2 beschrieben. Eine Strahlungsquelle, insbesondere eine Infrarotstrahlungsquelle, beleuchtet den Bereich, in dem dreidimensionale Raumkoordinaten zu erfassen sind, und die von den Markern reflektierte Strahlung wird mit Hilfe von zumindest zwei Kameras aus jeweils unterschiedlichen Blickwinkeln erfasst. Alternativ können die zu referenzierenden Gegenstände Strahlung, insbesondere Infrarotstrahlung, auch aktiv abstrahlen, zu welchem Zweck an den Gegenständen Strahlungsquellen befestigt sind, beispielsweise LED's, der Strahlung wie zuvor beschrieben erfasst wird. Die Kamerasignale werden mit Hilfe einer nachgeordneten Rechnereinheit und mittels bekannter Algorithmen ausgewertet.

Für die Referenzierung ist es erforderlich, dass stets zumindest zwei Kameras den Bereich, in dem dreidimensionale Raumkoordinaten zu erfassen sind, aus unterschiedlichen Blickwinkeln überwachen, ohne dass das relevante Gesichtfeld der Kameras verdeckt ist. Dabei ist es wünschenswert, dass die zumindest zwei Kameras in möglichst einfacher Weise in Anpassung an die jeweiligen Gegebenheiten justiert werden können.

Die DE 196 39 615 C2 offenbart zu diesem Zweck zwei von einem gemeinsamen Rahmen gehaltene Kameras, die seitlich über der zu erfassenden Szene, beispielsweise Operationstisch, positioniert werden müssen. Dies kann in gewissen Situationen schwierig sein. So hängt in einem Operationssaal die geeignete Position der Kameras des Referenzierungssystems insbesondere davon ab, an welchen Positionen um den Operationstisch herum das Operationspersonal steht und wo Geräte, die das Blickfeld der Kameras verdecken könnten, positioniert sind. Dabei ist zu berücksichtigen, dass unterschiedliche Operationen unterschiedliche Geräte und Positionen erfordern können. Somit ist eine geeignete Positionierung und Ausrichtung der Kameras vergleichsweise mühsam.

Bei anderen Referenzierungssystemen werden die Kameras deshalb entweder von der Decke im Operationssaal abgehängt oder an anderen geeigneten Stellen im Operationssaal befestigt, beispielsweise am Computer selbst oder in Raumecken. Dennoch kann es ohne weiteres vorkommen, dass die Gesichtfelder von einer oder mehreren Kameras gleichzeitig verdeckt werden, was eine genaue Referenzierung unmöglich macht. Bei einer Positionierung der Kameras in vergleichsweise großem Abstand zu dem relevanten Bereich, in dem dreidimensionale Raumkoordinaten zu erfassen sind, besteht außerdem der Nachteil, dass vergleichsweise viel Leerinformation von den Kameras erfasst wird, was die Genauigkeit der Referenzierung beinträchtigen kann oder zumindest den Rechenaufwand hierfür unnötigerweise erhöht.

Die WO 99/27839 offenbart ein chirurgisches Positionierungssystem, bei welchem eine Infrarotlichtquelle zur Erzeugung von durch Marker reflektiertem Licht zusammen mit Kameras an einem gemeinsamen Träger angeordnet ist.

Aufgabe der vorliegenden Erfindung ist es, ein System zur Referenzierung von dreidimensionalen Raumkoordinaten, insbesondere von chirurgischen oder medizinischen Instrumenten, zu schaffen, das in noch einfacherer Weise zu bedienen und einzurichten ist.

Diese Aufgabe wird gelöst durch ein System zur kombinierten schattenfreien Ausleuchtung eines vorgebbaren Bereichs und zur gleichzeitigen Referenzierung von dreidimensionalen Raumkoordinaten mit den Merkmalen nach Patentanspruch 1. Vorteilhafte Weiterbildungen sind Gegenstand der rückbezogenen Unteransprüche.

Gemäß einem ersten Gesichtspunkt der vorliegenden Erfindung wird eine Kombination aus zwei an sich bekannten Einzelsystemen geschaffen, nämlich einer Operationslampe als Lichtquelle zur schattenfreien Beleuchtung eines interessierenden Bereichs, in welchem die dreidimensionalen Raumkoordinaten erfasst werden sollen, und einem Kamerasystem, das in an sich bekannter Weise zur Referenzierung von dreidimensionalen Raumkoordinaten ausgelegt ist, insbesondere zur Referenzierung von chirurgischen und medizinischen Instrumenten. Erfindungsgemäß werden die Lichtquelle und das Kamerasystem so miteinander kombiniert, dass Lichtquelle und Kameras so gemeinsam gehalten sind, dass die von den Kameras erfassten optischen Signale zur Referenzierung von dreidimensionalen Raumkoordinaten in einem Bereich auswertbar sind, der den von der Lichtquelle schattenfrei beleuchteten Bereich umfasst oder im Wesentlichen identisch mit diesem ist.

Bei der Lichtquelle zur schattenfreien Beleuchtung eines Bereichs handelt es sich um eine Lichtquelle, die in Operationssälen, aber auch in Arztpraxen oder Zahnarztpraxen Verwendung findet. Eine solche Lichtquelle kann ein Leuchtmittel oder eine Mehrzahl von Leuchtmitteln in üblicher Weise punktsymmetrischer Anordnung umfassen. Ganz besonders bevorzugt wird das Kamerasystem erfindungsgemäß mit einer üblichen Operationslampe kombiniert. Zur vorgebbaren schattenfreien Beleuchtung werden solche Lichtquellen üblicherweise von einer Tragarmkonstruktion mit mehreren Gelenken getragen, um den zu beleuchtenden Bereich von oberhalb zu beleuchten. Dabei kann durch Verstellen der Tragarmkonstruktion die Position und/oder Orientierung der Lichtquelle in Bezug auf den auszuleuchtenden Bereich beliebig verändert werden.

Der vorliegenden Erfindung liegt die Erkenntnis zugrunde, dass die Lichtquelle erfahrungsgemäß stets so positioniert und/oder orientiert wird, dass der zu beleuchtende Bereich optimal beleuchtet ist, was in aller Regel bedeutet, dass die Sichtlinie von der Lichtquelle zu den zu beleuchteten Bereich nicht versperrt ist, sei es durch störende Geräte oder durch Personal. Die vorliegende Erfindung bedient sich nun dieser im Wesentlichen unversperrten Sichtlinie in überraschend einfacher Weise und positioniert die Kameras des Referenzierungssystems innerhalb oder in der näheren Umgebung der Lichtquelle, wie nachfolgend ausgeführt, so dass auch die Kameras, die der Referenzierung dienen, ein unversperrtes Gesichtsfeld auf die interessierenden Bereiche haben. Die vorliegende Erfindung nutzt weiterhin den Umstand, dass normalerweise in dem von der Lichtquelle schattenfrei beleuchteten Bereich die relevanten Vorgänge erfolgen, für die dreidimensionale Raumkoordinaten erfasst werden sollen. Somit wird erfindungsgemäß in überraschend einfacher Weise eine im Wesentlichen nicht verdeckte schattenfreie Beleuchtung kombiniert mit einem im Wesentlichen nicht verdeckten Gesichtsfeld der Kamera des Referenzierungssystems.

Damit eine genaue Referenzierung möglich ist, überlappen die Gesichtsfelder der mindestens zwei Kameras zumindest in dem beleuchteten Bereich. Der Überlappungsbereich der Gesichtsfelder der Kameras kann jedoch auch größer sein als der beleuchtete Bereich, was insbesondere dann von Vorteil ist, wenn die Lichtquelle unabhängig von den Referenzierungskameras positioniert und orientiert werden kann. Die zumindest zwei Kameras überwachen somit den beleuchteten Bereich aus zumindest zwei unterschiedlichen Blickwinkeln, was mit Hilfe bekannter Algorithmen eine Erfassung von dreidimensionalen Raumkoordinaten in dem Überlappungsbereich ermöglicht.

Während die an und für sich aus dem Stand der Technik bekannten Einzelkomponenten, nämlich die Lichtquelle und das Kamerasystem, im Stand der Technik unabhängig voneinander vertrieben und installiert werden, ist die vorliegende Erfindung auf ein beide Einzelkomponenten umfassendes System gerichtet, bei dem die Einzelkomponenten so gemeinsam gehalten sind, dass sowohl eine schattenfreie Beleuchtung vorgebbarer Bereiche als auch eine Referenzierung von dreidimensionalen Raumkoordinaten in dem so ausgeleuchteten Bereich möglich ist. Grundsätzlich kann die gemeinsame Halterung so erfolgen, dass sowohl die Kameras als auch die Lichtquelle unabhängig voneinander von einer Decke, beispielsweise in einem Operationssaal, abgehängt sind. Gemäß einer bevorzugten Ausführungsform werden jedoch zumindest zwei Kameras des Referenzierungssystems und die Lichtquelle gemeinsam mittels einer mechanischen Halterung gehalten. Ganz besonders bevorzugt werden die zumindest zwei Kameras des Referenzierungssystems in unmittelbarer Nähe zu der Lichtquelle gemeinsam mit dieser gehalten. Da Lichtquellen zur schattenfreien Beleuchtung üblicherweise einen kreisförmigen oder elliptischen Querschnitt aufweisen, sind die zumindest zwei Kameras des Referenzierungssystems nahe des Umfangsrands der Lichtquelle angeordnet, ganz besonders bevorzugt im Wesentlichen in der von der Lichtquelle aufgespannten Ebene oder geringfügig aus dieser vorstehend. Grundsätzlich können die Kameras jedoch auch hinter der von der Lichtquelle aufgespannten Ebene angeordnet sein.

Um die Referenzierung zu erleichtern, sind die zumindest zwei Kameras so in unmittelbarer Nähe der Lichtquelle angeordnet, dass das jeweilige Gesichtsfeld der Kameras durch die Lichtquelle bzw. durch das Gehäuse der Lichtquelle nicht verdeckt ist. Da die Gesichtsfelder der Kameras in dem beleuchteten Bereich überlappen sollten, sind die Kameras üblicherweise relativ zur optischen Achse der Lichtquelle geneigt. Wenn die Kameras also hinter der von der Lichtquelle aufgespannten Ebene angeordnet sind, so sind die radial einwärts geneigten Kameras radial auswärts versetzt, so dass das Gesichtsfeld der Kameras nicht durch den Umfangsrand der Lichtquelle verdeckt ist.

Gemäß einer besonders bevorzugten Ausführungsform sind zumindest zwei Kameras, in das Gehäuse der Lichtquelle integriert und im Wesentlichen parallel zu der optischen Achse der Lichtquelle ausgerichtet. Dieser Ausführungsform liegt der Erkenntnis zugrunde, dass ein Chirurg oder behandelnder Arzt sich in aller Regel nicht so weit vorbeugt, dass die Lichtquelle verdeckt wird, sondern dass der Chirurg oder Arzt üblicherweise nur mit den Händen und medizinischen Geräten in dem beleuchteten Bereich hantiert. Vorteilhaft ist, dass die Referenzierungskameras automatisch optimal orientiert sind, weil ja der zu referenzierende Bereiche üblicherweise optimal ausgeleuchtet werden muss.

Bevorzugt sind die in die Lichtquelle integrierten Kameras in Abschnitten der Lichtquelle angeordnet, wo keine Leuchtmittel vorgesehen sind. Üblicherweise haben Operationslampen mehrere Leuchtmittel, die typischerweise punktsymmetrisch um die Mitte der Lichtquelle herum angeordnet sind. So können beispielsweise mehrere Strahler in punktsymmetrischer Anordnung in ein im Wesentlichen rundes Lampengehäuse integriert sein. Auf dem Boden des Lampengehäuses befinden sich Durchbrüche, die auch mit Glasfenstern abgedeckt sein können, durch die hindurch das Licht auf den Operationstisch emittiert wird. Somit gibt es auf dem Boden des Lampengehäuses auch nicht durchbrochene Bereiche, die für die Referenzierungskameras genutzt werden können. Bevorzugt sind in diesen Bereichen jeweils Sichtfenster für die Referenzierungskameras vorgesehen, die gleichfalls von einem Glasfenster oder dergleichen abgedeckt sein können, um die Konvektionsverhältnisse in der Operationslampe nicht zu verändern. Die Kameras können bei dieser Ausführungsform im Wesentlichen parallel zu der optischen Achse der Lichtquelle ausgerichtet sein, da der Abstand zwischen den Referenzierungskameras vergleichsweise gering gewählt werden kann. Der integrierte Aufbau ermöglicht in besonders einfacher Weise eine optimale Orientierung der Kameras. Gleichzeitig wird sichergestellt, dass stets dort, wo die Operationslampe eingesetzt wird auch Referenzierungskameras vorhanden sind. Eine Operation kann somit schneller gestartet werden, was insbesondere bei mobilen Operationslampen von besonderem Vorteil ist. Gleichzeitig werden die Referenzierungskameras von dem Gehäuse der Lichtquelle geschützt.

Natürlich können bei der vorgenannten Ausführungsform auch weitere Kameras außerhalb des Gehäuses der Lichtquelle angeordnet sein, beispielsweise in der vorstehend beschriebenen Art. Diese sind bevorzugt gemeinsam mit dem Lampengehäuse gehalten oder sind beispielsweise an dessen Umfangsrand befestigt. Die Paare von Kameras können auf einer gemeinsamen Achse fluchtend oder auf sich kreuzenden Achsen, beispielsweise auf zueinander orthogonalen Achsen, angeordnet sein.

Gemäß einer bevorzugten Ausführungsform sind die Referenzierungskameras starr mit der Lichtquelle verbunden. Wenn Position und/oder Orientierung der Kameras einmal korrekt eingestellt sind, brauchen die Kameras nicht weiter verstellt werden. Somit ist in optimaler Weise gewährleistet, dass die Kameras stets richtig positioniert und orientiert sind.

Gemäß einer weiteren Ausführungsform kann jedoch die Orientierung und/oder Positionierung von einer oder von zumindest zwei Kameras relativ zu der Lichtquelle verstellt werden, was eine noch flexiblere Anpassung an die jeweiligen Gegebenheiten ermöglicht, beispielsweise bei Verwendung besonders hoher oder sperriger Geräte in einem Operationssaal, im Falle von besonders großen Personen, die um einen Operationstisch stehen und dergleichen.

Grundsätzlich können für die Referenzierungskameras beliebige Anordnungen relativ zu der Lichtquelle vorgegeben werden. Gemäß einer bevorzugten Ausführungsform sind die Kameras jedoch im Wesentlichen punktsymmetrisch zur Mitte der Lichtquelle angeordnet. Ganz besonders bevorzugt sind jeweils zwei Kameras auf gegenüberliegenden Seiten der Lichtquelle angeordnet. Sind mehr als drei Kameras vorgesehen, so können diese beispielsweise punktsymmetrisch in Bezug auf die Mitte der Lichtquelle angeordnet sein.

Gemäß einer weiteren Ausführungsform sind jeweils einige Kameras, beispielsweise jeweils zwei, zu Gruppen von Kameras zusammengefasst, wobei die Kameras einer Gruppe mittels Verstellelementen gemeinsam verstellt werden können, um eine neue Position und/oder Orientierung relativ zu der Lichtquelle einzunehmen. Die Gruppen können auf einer gemeinsamen Achse fluchtend oder auf sich kreuzenden Achsen, beispielsweise auf zueinander orthogonalen Achsen, angeordnet sein.

Zu diesem Zweck können die Kameras einer Kameragruppe auf einer gemeinsamen mechanischen Verstellgruppe befestigt. Gemäß einer bevorzugten Ausführungsform werden die Neigungswinkel, um die die Kameras relativ zu der optischen Achse der Lichtquelle geneigt sind, gemeinsam verstellt, wobei die Kameras bevorzugt symmetrisch relativ zur optischen Achse der Lichtquelle geneigt sind. Sind beispielsweise zwei Kameras der Kameragruppe auf gegenüberliegenden Seiten einer im Wesentlichen runden Lichtquelle angeordnet, so werden die Neigungswinkel der Kameras bevorzugt gegenläufig in Bezug auf die Mitte der Lichtquelle verstellt, so dass in besonders vorteilhaft einfacher Weise der Überlappungsbereich der Kameras verstellt werden kann, wobei der Schwerpunkt des Überlappungsbereichs stets mit dem Schwerpunkt des von der Lichtquelle ausgeleuchteten Bereichs übereinstimmt.

Die vorgenannte gemeinsame Bewegbarkeit von Lichtquelle und Kameras schließt nicht aus, dass die Lichtquelle für besondere Zwecke nicht auch völlig unabhängig von den Kameras bewegbar ist, beispielsweise wenn ein Bereich seitlich ausgeleuchtet werden soll oder wenn eine weitere Lichtquelle hinzugeschaltet werden soll. Dem Fachmann werden beim Studium der nachfolgenden Beschreibung der bevorzugten Ausführungsformen geeignete mechanische Halterungen ohne weiteres erkennbar sein.

Zur Referenzierung werden üblicherweise Kameras eingesetzt, die optische Signale nur im infraroten Spektralbereich erfassen. Zu diesem Zweck wird üblicherweise das sichtbare Spektrum mit Hilfe von Infrarot-Transmissionsfiltern abgeblockt. Da einerseits hohe optische Ausgangsleistungen für die Lichtquelle erwünscht sind, insbesondere in Operationssälen, andererseits jedoch eine übermäßige Wärmebelastung des beleuchteten Bereichs vermieden werden soll, wird gemäß einer besonders bevorzugten Ausführungsform der infrarote Strahlungsanteil der Lichtquelle mit Hilfe von Filtern zumindest teilweise herausgefiltert. Somit kann eine störende Beeinträchtigung der optischen Signale, die von den Kameras erfasst werden, vermieden werden. Maßnahmen zur Filterung des Lichts der Lichtquelle sind dem Fachmann von Operationslampen bekannt.

Ganz besonders bevorzugt umfasst das erfindungsgemäße System zumindest drei Kameras zur Referenzierung. Wird das Gesichtsfeld einer Kamera verdeckt, beispielsweise durch Geräte oder Personen, so kann zur Erfassung optischer Signale auf eine andere Kamera ausgewichen werden, deren Gesichtsfeld nicht verdeckt ist. Um dieses Wechseln auf eine andere Kamera automatisch zu bewerkstelligen ist gemäß dieser bevorzugten Ausführungsform eine Auswerteschaltung vorgesehen, die die von den Kameras erfassten elektronischen Signale auzswertet. Eine Verdeckung des Gesichtsfelds der Kamera zeichnet sich häufig durch charakteristische Signale aus, auf die hin die Auswerteschaltung die erfassten Signale analysiert. Beispielsweise kann sich ein verdeckter Bereich durch eine plötzliche Intensitätsänderung des erfassten Bereichs bemerkbar machen. Werden solche charakteristischen Signale festgestellt, so leitet die Auswerteschaltung nicht mehr die elektrischen Signale der verdeckten Signale an den zur Referenzierung verwendeten Rechner weiter, sondern wechselt automatisch auf eine andere Kamera, für die anhand der charakteristischen Signale eine Verdeckung nicht detektiert wird.

Ein zweiter Gesichtspunkt der vorliegenden Erfindung ist gerichtet auf ein Referenzierungssystem, wie es beispielsweise aus der DE 196 39 615 C2 bekannt ist. Erfindungsgemäß zeichnet sich dieses System dadurch aus, dass die mindestens zwei Kameras und die Lichtquelle zum schattenfreien Beleuchten eines Bereichs so gemeinsam gehalten sind, dass die Signale zur Referenzierung von dreidimensionalen Raumkoordinaten in dem beleuchteten Bereich auswertbar sind. Zu diesem Zweck ist das Kamerasystem und die Lichtquelle gemäß den zuvor im Zusammenhang mit dem ersten Gesichtspunkt beschriebenen Ausführungsformen ausgestaltet. Das Referenzierungssystem kann in dem Sinne aktiv oder passiv arbeiten, als dass das Licht entweder von Lichtquellen, die an den zu referenzierenden Gegenständen befestigt sind, emittiert wird oder von Reflektoren, die an den zu referenzierenden Gegenständen befestigt sind, reflektiert wird.

Nachfolgend werden bevorzugte Ausführungsformen der vorliegenden Erfindung in beispielhafter Weise und unter Bezugnahme auf die beigefügten Zeichnungen beschrieben werden, worin:
- Figur 1a/1b: eine schematische Unteransicht eines Systems gemäß einer ersten Ausführungsform der vorliegenden Erfindung und einer Modifikation davon ist;
- Figur 2: ein Querschnitt durch die erste Ausführungsform gemäß Figur 1 ist;
- Figur 3: eine Seitenansicht einer zweiten Ausführungsform des Systems gemäß der vorliegenden Erfindung darstellt.

In den Figuren bezeichnen identische Bezugszeichen identische oder gleichwirkende Elemente oder Baugruppen.

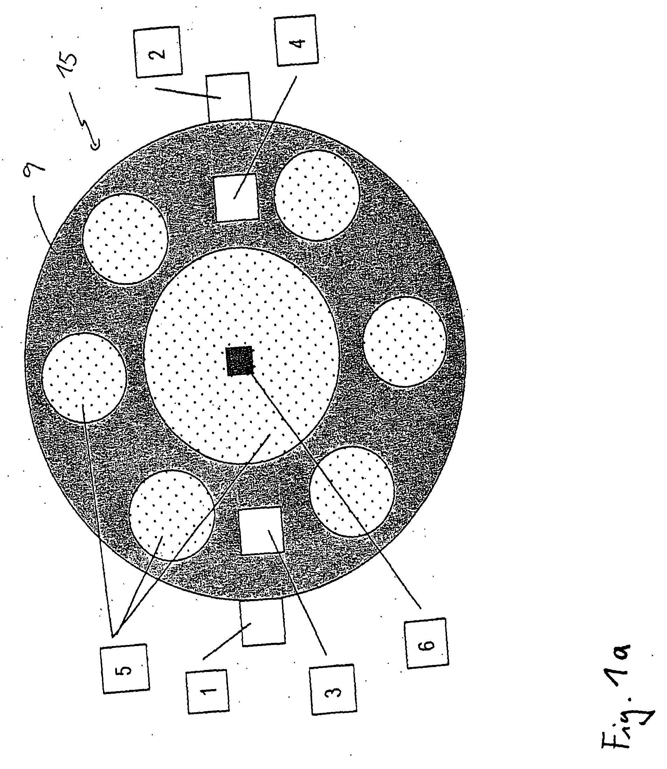

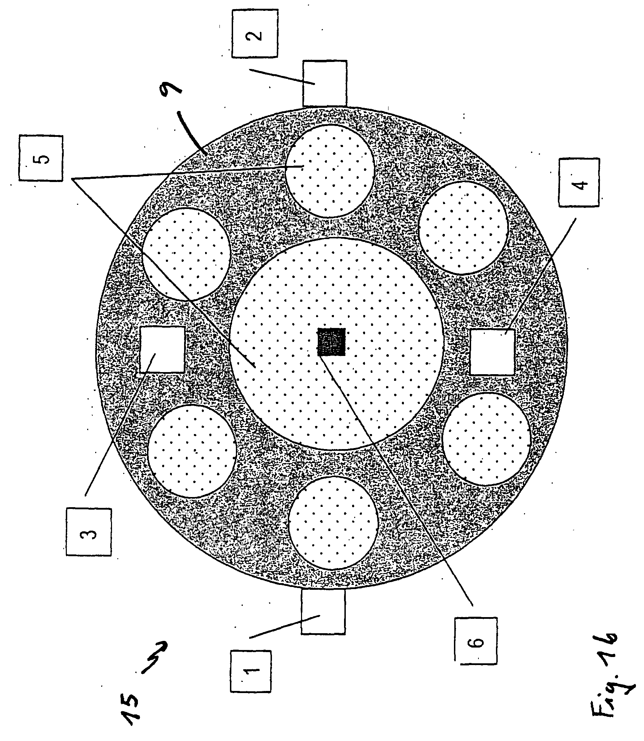

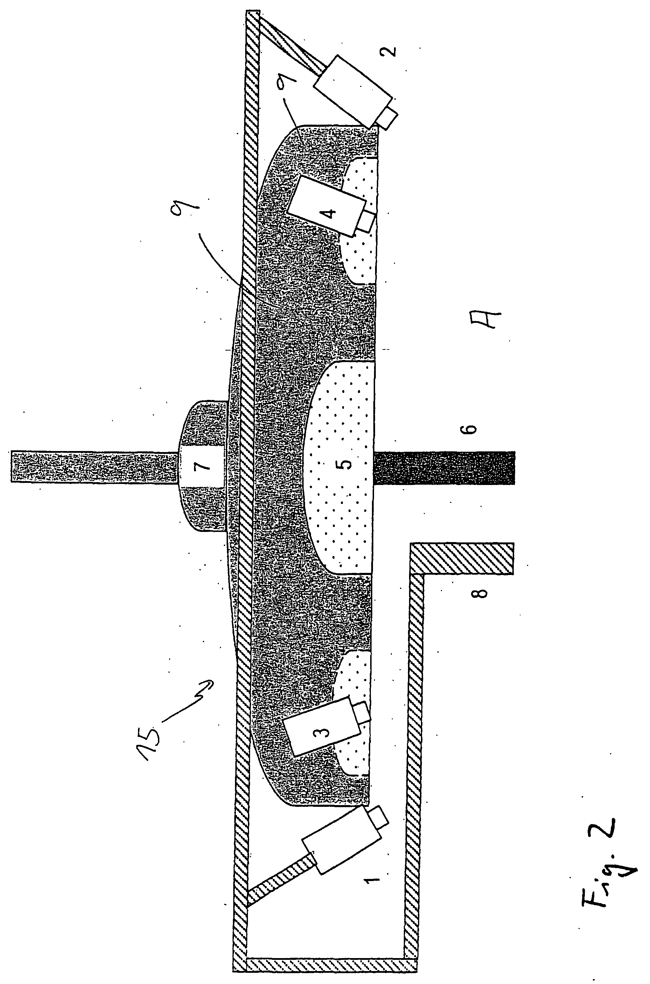

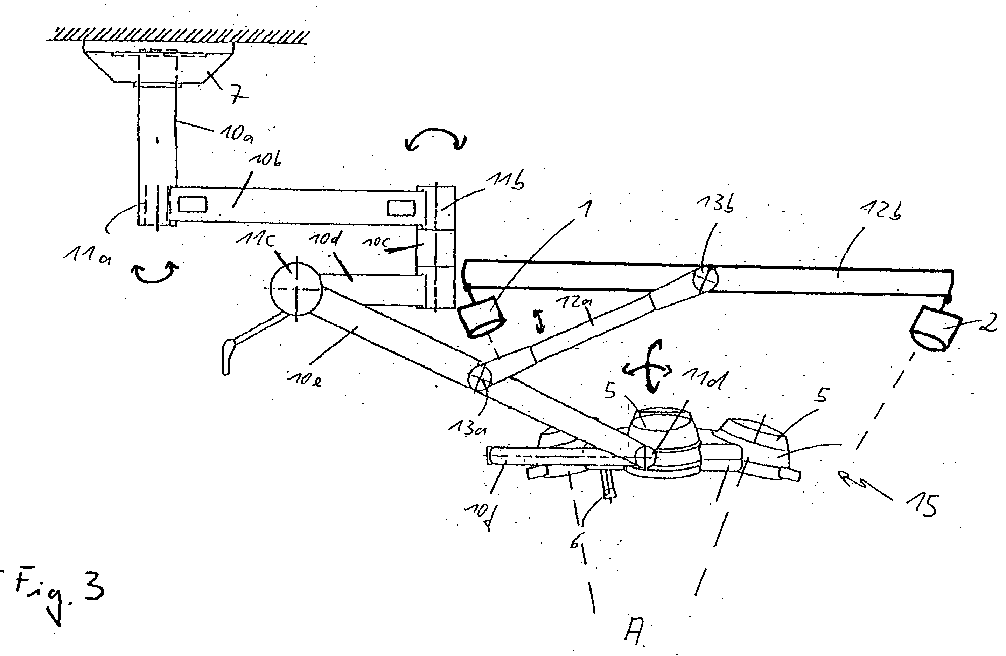

Die Figuren 1 und 2 zeigen schematisch in einer Unteransicht und im Querschnitt ein System gemäß einer ersten Ausführungsform der vorliegenden Erfindung. Das System umfasst die Lichtquelle 15 und vier Kameras 1 bis 4, die der Referenzierung von dreidimensionalen Raumkoordinaten gemäß dem in der DE 196 39 615 C2 beschriebenen optischen Navigierungsverfahren dienen. Zu diesem Zweck werden die von den Kameras 1 bis 4 erfassten Signale an eine nachgeordnete Rechnereinheit (nicht dargestellt) weitergeleitet. Die Lichtquelle 15 hat einen kreisförmigen Querschnitt und umfasst ein zentrales Leuchtmittel 5 und sechs dazu punktsymmetrisch angeordnete Leuchtmittel 5, die jeweils in dem Gehäuse 9 gehalten sind. Die Leuchtmittel 5 leuchten einen Bereich A im Wesentlichen schattenfrei aus. Zu diesem Zweck können die Leuchtmittel 5 das Licht im Wesentlichen senkrecht aus der von dem Boden des Gehäuses 9 aufgespannten Ebene ausstrahlen oder können um einen Winkel geneigt sein, was dem Fachmann auf diesem Gebiet bekannt ist. Üblicherweise wird die Lichtquelle 15 oberhalb des beleuchteten Bereichs A gehalten, zu welchem Zweck eine nicht dargestellte Deckenhalterung oder dergleichen vorgesehen ist, wie diese beispielhaft in der Figur 3 dargestellt ist. Mit Hilfe des zentral oder auch seitlich angeordneten Handgriffs 6 kann die Lichtquelle 15 relativ zu einem Objekt beliebig positioniert und/orientiert werden. Der Verstellung wirken ausreichende Kräfte entgegen, so dass die einmal eingenommene Position sich nicht wieder von alleine ändert.

Für Operationszwecke weist die Lichtquelle 15 üblicherweise einen vergleichsweise großen Durchmesser auf, beispielsweise im Bereich von 60 bis 80 cm. Leuchtstärke und Orientierung der Leuchtmittel sind dabei bevorzugt so gewählt, dass bei optimaler Ausleuchtung eines Bereichs ein üblicher Arbeitsabstand gewährleistet ist, beispielsweise in einem Abstand von etwa 107 cm bzw. in einem Bereich zwischen etwa 91 und etwa 123 cm Abstand zwischen Lichtquelle und auszuleuchtendem Arbeitsbereich.

Der Infrarotanteil des von den Leuchtmitteln 15 emittierten Lichts kann zumindest teilweise herausgefiltert sein, zu welchem Zweck in den in Figur 1 ersichtlichen Durchbrüchen im Boden des Lampengehäuses 9 geeignete optische Elemente vorgesehen sind, beispielsweise dichroitische Reflektoren, die abhängig von der Wellenlänge den infraroten Spektralanteil mit einem nicht dargestellten Wärmeabsorbierer reflektieren, während der sichtbare Spektralanteil durch die Durchbrüche in dem Lampengehäuse 9 hindurch gelassen wird. Selbstverständlich können die Durchbrüche mit Abdeckfenstern abgedeckt sein. In dem Boden des Lampengehäuses 9 können auch zusätzliche nicht dargestellte Konvektionsöffnungen vorgesehen sein, so dass eine die Lampe durchstreichende Luftströmung eine Überhitzung der Komponenten im Lampeninneren verhindert.

In unmittelbarer Nähe des Umfangsrands der Lichtquelle 15 sind zwei Kameras 1, 2 angeordnet, die relativ zu der durch den Handgriff 6 verlaufenden optischen Achse der Lichtquelle 15 einwärts geneigt sind, wie in der Figur 2 gezeigt. Die Kameras 1, 2 können unmittelbar an dem Außenumfangsrand des Lampengehäuses 9 montiert sein oder an einem gemeinsamen Arm, der entweder an dem Lampengehäuse 9 selbst oder in unmittelbarer Nähe des Lampengehäuses 9 befestigt ist. Zu diesem Zweck kann der nicht dargestellte Haltearm auch von der Decke abgehängt sein. Wie in der Figur 2 ersichtlich, ist das Gesichtsfeld der Kameras 1,2 nicht von Abschnitten des Lampengehäuses 9 verdeckt. Die Gesichtsfelder der Kameras 1, 2 überlappen in dem Bereich A, was eine stereoskopische Raumerfassung und somit eine Erfassung von dreidimensionalen Raumkoordinaten zumindest in dem Bereich A ermöglicht. Die Längsachsen der Kameras 1, 2 fluchten im Wesentlichen mit der optischen Achse der Lichtquelle 15. Das heißt die von den Längsachsen der Kameras 1, 2 aufgespannte Ebene beinhaltet, die im Wesentlichen durch den Handgriff 6 verlaufende optische Achse der Lichtquelle 15 oder ist in der Nähe von dieser angeordnet.

In das Lampeninnere integriert sind zwei weitere Kameras 3, 4, die den Bereich A durch Aussparungen in dem Boden des Lampengehäuses 9 hindurch überwachen. In den Aussparungen können Sichtfenster und/oder Infrarotfilter angebracht sein. Wie in der Figur 2 ersichtlich, sind auch die Kameras 3, 4 relativ zu der optischen Achse der Lichtquelle 15 geneigt und überlappen zumindest in dem Bereich A, um dort eine stereoskopische Erfassung und somit eine Referenzierung von dreidimensionalen Raumkoordinaten zu ermöglichen.

Aufgrund der gemeinsamen Halterung der Kameras 1 bis 4 mit der Lichtquelle 15 sind die Kameras automatisch so orientiert, dass der von der Lichtquelle 15 ausgeleuchtete Bereich A auch referenziert werden kann.

Natürlich können die am äußeren Umfangsrand des Lampengehäuses 9 angeordneten Kameras 1, 2 auch oberhalb der Lichtquelle angeordnet sein. Damit die Gesichtsfelder dieser Kameras nicht durch das Lampengehäuse 9 verdeckt sind, sind die Kameras 1, 2 radial auswärts versetzt angeordnet, und zwar außerhalb eines Kegelstumpfes, dessen Begrenzungsflächen im Wesentlichen durch die Längsachsen der in der Figur 2 dargestellten Kameras 1, 2 vorgegeben ist.

Grundsätzlich können zumindest die Kameras 1, 2 auch unabhängig von der Lichtquelle 15 positioniert und/oder orientiert werden. Zu diesem Zweck ist der in der Figur 2 schematisch dargestellte zusätzliche Handgriff 8 vorgesehen, der grundsätzlich auch in den Handgriff 6 integriert sein kann. Durch Verstellung des Handgriffes 8 werden geeignete Positionierungsund Orientierungsbewegungen zumindest der Kameras 1, 2, gegebenenfalls auch der Kameras 3, 4, ausgelöst. Beispielsweise kann der Neigungswinkel der Kameras relativ zu der optischen Achse der Lichtquelle 15 geändert werden, kann der Neigungswinkel der Kameras senkrecht zu der Zeichnungsebene der Figur 2 geändert werden und kann die radiale Position der Kameras variiert werden. Die Verstellung kann manuell mit Hilfe des Handgriffs 8 erfolgen. Selbstverständlich können auch eines oder mehrere motorische Verstellelemente vorgesehen sein, beispielsweise durch den Handgriff 8 einzeln oder gruppenweise betätigbare elektrische Verstellmotoren. Die Verstellmotoren können drahtlos oder drahtgebunden mit Steuerbefehlen versorgt werden.

Geeignete Halterungen für die Kameras werden dem Fachmann auf diesem Gebiet beim Studium dieser Patentbeschreibung ohne weiteres ersichtlich sein. Die Verstellbarkeit richtet sich nach den Erfordernissen und kann eine axiale Verschiebung und/oder eine Verdrehung um die Längsachse der Kameras und/oder eine Verschwenkung um die Kameralängsachse in eine oder zwei Raumrichtungen und so weiter umfassen.

Während in der Figur 1a die beiden Gruppen von Kameras 1, 2 und 3, 4 auf einer gemeinsamen Achse fluchtend angeordnet sind, sind in der Figur 1b die beiden Gruppen von Kameras auf zueinander orthogonalen Achsen, die sich in der Lichtquellenmitte kreuzen, angeordnet.

Die Figur 3 zeigt in einer Seitenansicht schematisch eine zweite Ausführungsform des erfindungsgemäßen Systems. Dieses umfasst die Lichtquelle 15 und die Kameras 1, 2, die gemeinsam von einer Tragarmkonstruktion von der Decke abgehängt sind. Ausgehend von der Deckenbefestigung 7 umfasst die Tragarmkonstruktion mehrere Arme 10a-f, die an den Gelenken 11a, 11b axial verdrehbar und in den Gelenkbereichen 11c und 11b senkrecht zur zweiten Ebene der Figur 3 verschwenkbar sind, so dass innerhalb der Reichweite der Arme 10a-f eine nahezu beliebige Positionierung und Orientierung der Lichtquelle 15 in Bezug auf ein zu beleuchtendes Objekt erzielt werden kann. Die Lichtquelle 15 selbst umfasst drei Leuchtmittel 5, die in sternförmiger, punktsymmetrischer Anordnung den Bereich A ausleuchten. Die Kameras 1, 2 werden von dem gemeinsamen Arm 12a, 12b der Kameraaufhängung 12a, 12b gehalten. Die Arme 12a, 12b sind in der dargestellten Weise um die Gelenke 13a, 13b senkrecht zu der Zeichenebene der Figur 3 verschwenkbar. Selbstverständlich kann statt der schwenkbaren Halterung auch eine relativ zu der Lichtquelle 15 starre Kameraaufhängung vorgesehen sein. Der Abstand des Arms 12b relativ zur Lichtquelle 15 ist vorzugsweise gering, beispielsweise im Bereich von etwa 5 bis etwa 50 cm, vorzugsweise im Bereich von etwa 5 bis etwa 30 cm und noch bevorzugter im Bereich von etwa 5 bis etwa 15 cm.

Wie in Figur 3 gezeigt, sind die Kameras 1, 2 radial einwärts geneigt, so dass deren Gesichtsfeld zumindest in dem ausgeleuchteten Bereich A überlappt, um eine 3D-Referenzierung zu ermöglichen.

Durch Verschwenken der Arme 12a, 12b der Kameraaufhängung und/oder Verstellen von nicht dargestellten Verstellmotoren können die Gesichtsfelder der Kameras 1, 2 geeignet geändert werden.

In der Lichtquelle 15 kann ein Erfassungsmittel vorgesehen sein, das aus der Orientierung der Lichtquelle 15 auf die Position des ausgeleuchteten Bereichs A schließt. An der Kameraaufhängung 12a, 12b ihrerseits kann ein Erfassungsmittel vorgesehen sein, das die Lagebeziehung der Kameras 1, 2 in Bezug auf die Lichtquelle 15 und somit in Bezug auf den ausgeleuchteten Bereich A automatisch erfasst. Bei Verstellen der Lichtquelle 15 und somit bei Veränderung des ausgeleuchteten Bereichs A wird von einer nicht dargestellten Rechnereinheit berechnet, ob der von den Kameras 1, 2 erfasste Bereich, in dem eine Referenzierung möglich ist (der oben genannte Überlappungsbereich), den aktuell ausgeleuchteten Bereich A mit umfasst. Ist dies nicht der Fall, so wird ein Steuersignal berechnet, das zur motorischen Nachführung der Kameras 1, 2 verwendet werden kann.

Wie in der Figur 3 gezeigt, sind die Kameras 1, 2 so orientiert, dass deren Gesichtsfelder durch die Lichtquelle 15 nicht oder nur unwesentlich verdeckt sind. Grundsätzlich können die Kameras 1, 2 in der oben beschriebenen Weise auch automatisch motorisch verstellt werden, sollte das Gesichtsfeld der Kameras durch die Lichtquelle 15 verdeckt sein. Hierzu muss dem oben genannten Rechnermittel Information betreffend die Außenabmessungen der Lichtquelle zur Verfügung stehen.

Die Kameras sind üblicherweise CCD-Videokameras, so dass die erfassten optischen Signale in digitaler Form für eine Auswertung zur Verfügung stehen. Anhand von charakteristischen Signaländerungen, beispielsweise plötzlichen Intensitätsänderungen, kann somit festgestellt werden, ob das Gesichtsfeld einer Kamera plötzlich verdeckt wird. Um stets eine geeignete Referenzierung sicherzustellen, ist eine nicht dargestellte Auswerteschaltung vorgesehen, die die jeweiligen elektrischen Signale der Kameras 1-4 untersucht. Wird eine charakteristische Signaländerung, die auf eine Verdeckung des Gesichtsfelds der Kamera schließen lässt, detektiert, so wechselt die Auswerteschaltung automatisch auf eine andere der Kameras 1-4 und leitet deren Signal an die Rechnereinheit des Referenzierungssystems weiter. Die elektronischen Signale der Kameras 1-4 können auch periodisch von der Rechnereinheit in einem vorbestimmten Schema daraufhin geprüft werden, ob das jeweilige Gesichtsfeld gerade verdeckt ist. Dann kann die Rechnereinheit nach einem vorgegebenen Algorithmus festlegen, welche der Kameras 1-4 zur Referenzierung verwendet werden sollen. Aufgrund der gemeinsamen Halterung von Kamerasystem und Lichtquelle können die zum Betreiben von Lichtquelle 15 und Videokameras 1-4 erforderlichen Anschlüsse als gemeinsames Anschlusskabel aus der Deckebefestigung 7 herausgeführt werden.

Grundsätzlich können die einzelnen Komponenten für das beanspruchte System auch einzeln vertrieben werden. Gemäß einer bevorzugten Ausführungsform zeichnet sich das erfindungsgemäße System insbesondere dadurch aus, dass Lichtquelle und Videokameras so gemeinsam gehalten sind, dass eine Referenzierung dreidimensionaler Raumkoordinaten in dem von der Lichtquelle 15 gerade ausgeleuchteten Bereich möglich ist. Zu diesem Zweck sind die Kameras insbesondere in unmittelbarer Nähe der Lichtquelle 15 angeordnet.

## Patentansprüche

1. System zum kombinierten Beleuchten eines vorgebbaren Bereichs (A) und zur Referenzierung von dreidimensionalen Raumkoordinaten, insbesondere von chirurgischen oder medizinischen Instrumenten, umfassend:
eine Operationslampe (15) zur Beleuchtung des Bereichs,
eine Strahlungsquelle zum Emittieren von Infrarotstrahlung, und
zumindest zwei Kameras (1-4) zur Erfassung von optischen Infrarotsignalen,
wobei die Operationslampe (15) und die Kameras (1-4) so gemeinsam gehalten sind, dass die optischen Infrarotsignale zur Referenzierung von dreidimensionalen Raumkoordinaten in dem beleuchteten Bereich (A) auswertbar sind.

2. System nach Anspruch 1, bei dem zumindest zwei Kameras (1, 2) außerhalb eines Gehäuses (9) der Operationslampe nahe eines Umfangsrandes des Gehäuses so angeordnet sind, dass die Sicht der Kameras auf den beleuchteten Bereich (A) durch Gehäuseabschnitte nicht verdeckt ist.

3. System nach Anspruch 1 oder 2, bei dem zumindest zwei Kameras (3, 4) in ein Gehäuse (9) der Operationslampe integriert sind, wobei jeweils Sichtfenster für die Kameras an Abschnitten des Gehäuses vorgesehen sind, wo keine Leuchtmittel (5) der Operationslampe (15) angeordnet sind.

4. System nach einem der vorhergehenden Ansprüche, bei dem die Operationslampe (15) und zumindest zwei Kameras starr miteinander verbunden sind, so dass die Operationslampe und die Kameras gemeinsam bewegbar sind.

5. System nach einem der Ansprüche 1 bis 3, bei dem die Orientierung und/oder Position von zumindest zwei Kameras relativ zur Operationslampe (15) einstellbar ist.

6. System nach Anspruch 5, bei dem zumindest zwei auf jeweils gegenüberliegenden Seiten der Operationslampe (15) angeordnete Kameras (1, 2; 3, 4) gemeinsam bewegbar sind, wobei ein Neigungswinkel der Kameras gegenläufig in Bezug auf eine Mitte der Operationslampe verstellbar ist.

7. System nach einem der vorhergehenden Ansprüche, bei dem zumindest zwei Kameras im Wesentlichen punktsymmetrisch zur Mitte der Operationslampe (15) angeordnet sind.

8. System nach einem der vorhergehenden Ansprüche, bei dem die Kameras mit Infrarot-Transmissionsfiltern versehen sind, um von Reflektoren reflektierte oder von Lichtquellen emittierte Infrarotstrahlung zu erfassen, wobei der Infrarotanteil des von der Operationslampe (15) emittierten Lichts zumindest teilweise herausgefiltert wird.

9. System nach einem der vorhergehenden Ansprüche, das zumindest drei Kameras umfasst, wobei eine Auswerteschaltung vorgesehen ist, um die von den Kameras erfassten Signale auszuwerten, um zu erfassen, wenn das Gesichtsfeld von zumindest einer Kamera verdeckt wird, und um zur Referenzierung von der jeweiligen Kamera mit dem verdeckten Gesichtsfeld auf eine andere Kamera der Mehrzahl von Kameras zu wechseln, deren Gesichtfeld nicht verdeckt ist.

## Claims

1. A system for the combined illumination of a pre-definable area (A) and for referencing three-dimensional spatial co-ordinates, in particular of surgical or medical instruments, comprising:
a operation lamp (15) for illuminating said area;
a radiation source for emitting infrared radiation; and
at least two cameras (1-4) for detecting optical infrared signals,
wherein said operation lamp (15) and said cameras (1-4) are held together such that said optical infrared signals may be evaluated, for referencing three-dimensional spatial co-ordinates in said illuminated area (A).

2. The system as set forth in claim 1, wherein at least two cameras (1,2) are arranged outside a casing (9) of the operation lamp and near a circumferential edge of said casing, such that the sight of said cameras onto said illuminated area (A) is not obstructed by sections of said casing.

3. The system as set forth in claim 1 or 2, wherein at least two cameras (3, 4) are integrated in said casing (9) of said operation lamp, viewing windows being respectively provided for said cameras on sections of said casing where no illuminating means (5) of said operation lamp (15) are arranged.

4. The system as set forth in any one of the preceding claims, wherein said operation lamp (15) and at least two cameras are rigidly connected to each other such that said operation lamp and said cameras may be moved together.

5. The system as set forth in any one of claims 1 to 3, wherein the orientation and/or position of at least two cameras may be adjusted relative to said operation lamp (15).

6. The system as set forth in claim 5, wherein at least two cameras (1, 2; 3, 4) arranged respectively on opposite sides of said operation lamp (15) may be moved together, an inclination angle of said cameras being adjustable, in the opposite direction, with respect to the centre of said operation lamp.

7. The system as set forth in any one of the preceding claims, wherein at least two cameras are arranged substantially point-symmetrically with respect to said centre of said operation lamp (15).

8. The system as set forth in any one of the preceding claims, wherein said cameras are provided with infrared transmission filters in order to detect infrared radiation reflected by reflectors or emitted by light sources, the infrared portion of said light emitted from said operation lamp (15) being at least in part filtered out.

9. The system as set forth in any one of the preceding claims, comprising at least three cameras, wherein an evaluation circuit is provided to evaluate the signals detected by said cameras, in order to detect when the field of view of at least one camera is obstructed, and in order - for referencing - to change from said respective camera with the obstructed field of view to another camera of said plurality of cameras, whose field of view is not obstructed.

## Revendications

1. Système combiné d'éclairage d'une zone (A) prédéterminable et de référencement de coordonnées dans l'espace à trois dimensions, en particulier d'instruments chirurgicaux ou médicaux, qui comprend:
une lampe d'opération (15) qui éclaire la zone,
une source de rayonnement qui émet un rayonnement infrarouge et
au moins deux caméras (1-4) qui détectent des signaux optiques infrarouge,
la lampe d'opération (15) et les caméras (1 à 4) étant maintenues ensemble de telle sorte que les signaux optiques infrarouges puissent être évalués pour référencer des coordonnées dans l'espace à trois dimensions dans la zone éclairée (A).

2. Système selon la revendication 1, dans lequel au moins deux caméras (1, 2) sont disposées à l'extérieur d'un boîtier (9) de la lampe d'opération à proximité d'un bord périphérique du boîtier de telle sorte que la vue des caméras sur la zone éclairée (A) ne soit pas recouverte par les parties du boîtier.

3. Système selon les revendications 1 ou 2, dans lequel au moins deux caméras (3, 4) sont intégrées dans un boîtier (9) de la lampe d'opération, des hublots étant prévus pour chacune des caméras dans des parties du boîtier dans lesquelles aucun moyen d'éclairage (5) de la lampe d'opération (15) n'est disposé.

4. Système selon l'une des revendications précédentes, dans lequel la lampe d'opération (15) et au moins deux caméras sont reliées rigidement l'une à l'autre de telle sorte que la lampe d'opération et les caméras puissent être déplacées ensemble.

5. Système selon l'une des revendications 1 à 3, dans lequel l'orientation et/ou la position d'au moins deux caméras par rapport à la lampe d'opération (15) sont ajustables.

6. Système selon la revendication 5, dans lequel au moins deux caméras (1, 2; 3, 4) disposées sur des côtés opposés de la lampe d'opération (15) peuvent être déplacées ensemble, un angle d'inclinaison des caméras pouvant être ajusté dans les deux sens par rapport au centre de la lampe d'opération.

7. Système selon l'une des revendications précédentes, dans lequel au moins deux caméras sont disposées essentiellement à symétrie centrale par rapport au milieu de la lampe d'opération (15).

8. Système selon l'une des revendications précédentes, dans lequel les caméras sont dotées de filtres de transmission infrarouges qui détectent le rayonnement infrarouge réfléchi par des réflecteurs ou émis par des sources de lumière, la portion infrarouge de la lumière émise par la lampe d'opération (15) étant filtrée au moins en partie.

9. Système selon l'une des revendications précédentes, qui comprend au moins trois caméras, un système d'évaluation étant prévu pour évaluer les signaux détectés par les caméras pour détecter quand le champ de vision d'au moins une caméra est recouvert et pour, en vue du référencement, passer de la caméra dont le champ de vision est recouvert à une autre caméra de la pluralité de caméras dont le champ de vision n'est pas recouvert.
